(19) Europäisches Patentamt
European Patent Office
Office européen des brevets

(11) **EP 1 067 105 B1**

(12) **EUROPÄISCHE PATENTSCHRIFT**

(45) Veröffentlichungstag und Bekanntmachung des Hinweises auf die Patenterteilung:
**11.09.2002   Patentblatt 2002/37**

(51) Int Cl.[7]: **C07C 19/01**, C07C 17/361

(21) Anmeldenummer: **00108356.7**

(22) Anmeldetag: **15.04.2000**

(54) **Verfahren zur Herstellung von 1,3-Dichlorpropan**

Process for the preparation of 1,3-dichloropropane

Procédé pour la préparation du dichloropropane- 1,3

(84) Benannte Vertragsstaaten:
**CH DE FR GB IT LI**

(30) Priorität: **09.06.1999   DE 19926165**

(43) Veröffentlichungstag der Anmeldung:
**10.01.2001   Patentblatt 2001/02**

(73) Patentinhaber: **Degussa AG**
**40474 Düsseldorf (DE)**

(72) Erfinder: **Kaufhold, Manfred, Dr.**
**45770 Marl (DE)**

(56) Entgegenhaltungen:
WO-A-97/44302          DE-A- 19 606 549
DE-C- 859 734          US-A- 2 084 710

• CHEMICAL ABSTRACTS, vol. 110, no. 7, 13. Februar 1989 (1989-02-13) Columbus, Ohio, US; abstract no. 57108k, MYSZKOWSKI J ET AL: "Process for manufacturing 1,3-dichloropropane" Seite 635; Spalte 1; XP002901372 & PL 138 136 B 31. Dezember 1988 (1988-12-31)
• CHEMICAL ABSTRACTS, vol. 109, no. 9, 29. August 1988 (1988-08-29) Columbus, Ohio, US; abstract no. 72980m, MULLAKHMETOVA Z F: "Reactions of 1,3-dioxacycloalkanes with halosilanes" Seite 647; Spalte 1; XP002901373 & ZH., KHIM, 1987,

**Beschreibung**

Die Erfindung betrifft ein Verfahren zur Herstellung von 1,3-Dichlorpropan durch Umsetzung von Bis(3-hydroxypropyl)ether mit Chlorwasserstoff in Gegenwart von tertiären basischen Stickstoffverbindungen Abdestillation des 1,3-Dichlorpropans und des Reaktionswassers und Aufarbeitung der beiden Phasen.

1,3-Dichlorpropan ist ein wichtiges Zwischenprodukt zur Herstellung von Pharma- und Agrochemikalien und dient auch als wenig giftiges Lösemittel. Synthesen von 1,3-Dichlorpropan sind seit langem aus der Literatur bekannt. Die meisten gehen vom Propandiol-1,3 aus und setzen dieses mit Thionylchlorid oder Phosphorpentachlorid um (siehe Clark Streight, Trans. roy. Soc. Canada (3) 23, 3 (1929) 77).

Nachteilig bei diesen Verfahren ist der relativ hohe Preis für den Einsatzstoff Propandiol-1,3. Es besteht aber ein großes Interesse an einem Verfahren, das mit einem wohlfeilen Einsatzstoff wirtschaftlich arbeitet und das technisch so einfach wie die üblichen Verfahren zur Herstellung von Chlorverbindungen aus den entsprechenden Hydroxylverbindungen zu realisieren ist.

Aufgabe der Erfindung war es daher, einen geeigneten Einsatzstoff für die Synthese von 1,3-Dichlorpropan zu finden, der wohlfeil ist und in ausreichender Menge zur Verfügung steht.

Die Aufgabe wird erfindungsgemäß dadurch gelöst, daß Bis(3-hydroxypropyl)ether als Ausgangsmaterial verwendet wird. Dieses Etherdiol fällt bei der Produktion von Propandiol-1,3 als Nebenprodukt an und kann nach EP-A-0 577 972 nur durch einen speziellen aufwendigen Verfahrensschritt gespalten werden.

Überraschenderweise wurde nun jedoch gefunden, daß unter Bedingungen, bei denen die Hydroxylgruppen des Etherdiols gegen Chloratome ausgetauscht werden, auch die Ethergruppe gespalten wird und daß aus 1 Mol Etherdiol und 4 Mol Chlorwasserstoff 2 Mol 1,3-Dichlorpropan und 3 Mol Wasser entstehen nach der Gleichung

$$HOCH_2CH_2CH_2OCH_2CH_2CH_2OH + 4\ HCl \rightarrow 2\ ClCH_2CH_2CH_2Cl + 3\ H_2O$$

Gegenstand der Erfindung ist daher ein Verfahren zur Herstellung von 1,3-Dichlorpropan, dadurch gekennzeichnet, daß man Bis(3-hydroxypropyl)ether in Gegenwart von tertiären basischen Stickstoffverbindungen mit Chlorwasserstoff umsetzt, das entstehende 1,3-Dichlorpropan und Reaktionswasser abdestilliert und beide Phasen aufarbeitet.

Als Zwischenprodukt tritt 3-Chlorpropanol auf. Der Bis(3-chlorpropyl)ether entsteht in geringer Menge in Abhängigkeit von den Destillationsbedingungen bei der Umsetzung. Er kann in die Reaktion zurückgeführt werden. Die leichte Spaltbarkeit des Ethers mit Chlorwasserstoff steht im Gegensatz zu Literaturangaben: so wird im "Houben-Weyl" (Band Halogenverbindungen, Georg Thieme Verlag Stuttgart, 1962, Seiten 839 und 840) ausgesagt, daß sich cyclische Ether wie z. B. Tetrahydrofuran und Allylether relativ leicht spalten lassen, daß sich (aber) die gesättigten aliphatischen Ether nur unter extremen Bedingungen mit Chlorwasserstoff in die Chloride verwandeln lassen. Weiterhin heißt es in der Monographie "Organisch-Chemische Experimentierkunst" von Weygand/Hilgetag (Johann Ambrosius Barth Verlag Leipzig, 1970) auf Seite 241 zur Etherspaltung mit Halogenwasserstoffen: "Von den Halogenwasserstoffen ist HJ besonders wirksam, HBr weniger, HCl am wenigsten". Danach war also anzunehmen, daß das Etherdiol mit Chlorwasserstoff kaum gespalten wird.

Das erfindungsgemäße Verfahren benötigt üblicherweise keine Lösemittel. Wenn es aus irgendwelchen Gründen jedoch vorteilhaft sein sollte, kann man als Lösemittel z. B. aliphatische oder aromatische Kohlenwasserstoffe, Halogenverbindungen und aliphatische Ether einsetzen.

Als Katalysatoren wirkende tertiäre basische Stickstoffverbindungen werden vorteilhaft in einer solchen Menge eingesetzt, daß sie die Funktion eines Lösemittels übernehmen.

Geeignete tertiäre basische Stickstoffverbindungen sind Pyridin und Alkylpyridine wie Methyl-, Dimethyl- und Ethylpyridin sowie technische Gemische von diesen Verbindungen (sog. Pyridinbasen), ferner Chinolin und Derivate des Chinolins, wie z. B. 2-Methylchinolin (Chinaldin) und 4-Methylchinolin (Lepidin), N,N-Dialkylanilin wie N,N-Dimethylanilin und tertiäre Amine wie Trialkylamine und Gemische der vorgenannten Verbindungen. Bevorzugt werden die sogenannten Pyridinbasen eingesetzt. Diese liegen bei und nach der Reaktion als Hydrochloride vor und sind in dieser Form leicht abtrenn- und mehrmals hintereinander einsetzbar.

Bei kontinuierlicher Fahrweise werden Etherdiol und Chlorwasserstoff gleichzeitig in den Reaktor zu den als Hydrochloride vorliegenden Pyridinbasen gegeben und 1,3-Dichlorpropan und Wasser abdestilliert.

In Gegenwart von tertiären basischen Stickstoffverbindungen, ausgeführt am Beispiel der Pyridinbasen, kann wie folgt vorgegangen werden:

Ein Pyridinbasegemisch wird vorgelegt und Chlorwasserstoff bis zur Sättigung eingeleitet. Dann wird das Etherdiol zugegeben und unter Einleiten von Chlorwasserstoff von ca. 20 °C auf ca. 120 °C erwärmt. Das Verhältnis Pyridinbase zu Etherdiol beträgt 0,1 Mol zu 5 Mol, vorzugsweise 0,5 bis 1,5 Mol zu 2 Mol. Die Menge an Chlorwasserstoff richtet sich nach dessen Aufnahmegeschwindigkeit. Er wird so schnell eingeleitet, daß sich immer ein kleiner Überschuß im Reaktor befindet. Damit die Reaktion schnell genug weitergeht, wird im Laufe der Zeit die Temperatur bis auf ca. 190 °C, bevorzugt bis auf 160 °C, erhöht. Während der Reaktion destilliert ständig ein Gemisch aus 1,3-Dichlorpropan und Wasser ab. Dieses Gemisch wird in an sich bekannter Weise aufgearbeitet und das Dichlorpropan durch Destillation gereinigt.

Die nur zum Teil umgesetzten Produkte 3-Chlorpropanol und Bis(3-chlorpropyl)ether können in die Reaktion zurückgeführt werden.

Das folgende Beispiel soll das erfindungsgemäße Verfahren näher erläutern, nicht jedoch auf die speziell genannten Umstände einschränken.

Beispiel

Es wurde eine Reaktionsapparatur aus Glas, die aus einem Vierhalskolben mit Rührer, Thermometer, Tropftrichter, Gaseinleitungsrohr und einer aufgesetzten Destillationsbrücke mit Vorlage bestand, verwendet.

In dem Kolben wurden 93,1 g Pyridinbase (technisches Gemisch aus Pyridin, Methylpyridin und Ethylpyridin) und 18,6 g Wasser vorgelegt.

In dieses Gemisch wurde gasförmiger Chlorwasserstoff bis zur Sättigung unter Kühlung bei Raumtemperatur eingeleitet. Durch den Wasserzusatz blieb die Lösung flüssig. Dann wurden 268,4 g (2 Mol) Bis(3-hydroxypropyl)ether bei Raumtemperatur zugegeben und weiter Chlorwasserstoff eingeleitet. Danach wurde unter weiterer Einleitung von Chlorwasserstoff in 0,5 Stunden bis auf 120 °C erwärmt. Bei dieser Temperatur wurde der Chlorwasserstoff schnell aufgenommen und es begann das Abdestillieren von Reaktionsprodukten und Wasser. Nach ca. 4 Stunden ließ die Chlorwasserstoffaufnahme etwas nach. Die Temperatur wurde innerhalb von 4 Stunden auf 160 °C erhöht. Nach insgesamt 19 Stunden war die Reaktion beendet.

Zu dem Destillat wurden 114 g Cyclohexan gegeben und damit Wasser ausgekreist. Danach wurde der Rückstand destillativ aufgearbeitet. Als Hauptlauf wurde 1,3-Dichlorpropan erhalten. Das Sumpfprodukt bestand aus 3-Chlorpropanol-1 und Bis(3-chlorpropyl)ether. Beide Produkte wurden in die Reaktion zurückgegeben und lieferten wie das Einsatzdiol (im nächsten Ansatz) weiteres 1,3-Dichlorpropan. Bei der Ausbeuteberechnung, die sich auf den Umsatz bezieht, werden deshalb diese Molmengen berücksichtigt.

Im Hauptlauf wurden 280,3 g 1,3-Dichlorpropan mit einer Reinheit von 99,1 % erhalten. Die Ausbeute bezogen auf den Einsatz betrug 61,4 %. Bei der Destillation fielen 24,0 g 3-Chlorpropanol-1 (100 %ig gerechnet) und 65,3 g Bis(3-chlorpropyl)ether (100 %ig gerechnet) an, die bei der Ausbeuteberechnung wegen ihres Wiedereinsatzes wie nicht umgesetztes Einsatzprodukt gewertet wurden. Hieraus läßt sich die Ausbeute an 1,3-Dichlorpropan bezogen auf (vollständigen) Umsatz zu ca. 85 % errechnen.

**Patentansprüche**

1. Verfahren zur Herstellung von 1,3-Dichlorpropan,
   **dadurch gekennzeichnet,**
   **dass** man Bis(3-hydroxypropyl)ether in Gegenwart von tertiären basischen Stickstoffverbindungen mit Chlorwasserstoff umsetzt, das entstehende 1,3-Dichlorpropan und Reaktionswasser abdestilliert und beide Phasen aufarbeitet.

2. Verfahren nach Anspruch 1,
   **dadurch gekennzeichnet,**
   **daß** die Umsetzung in Gegenwart von Pyridin, Alkylpyridinen, Chinolin, Chinolinderivaten, Dialkylanilinen oder Trialkylaminen oder deren Mischungen durchgeführt wird.

3. Verfahren nach Anspruch 1,
   **dadurch gekennzeichnet,**
   **daß** die Umsetzung bei einer Temperatur zwischen 20 °C und 190 °C stattfindet.

4. Verfahren nach Anspruch 1,
   **dadurch gekennzeichnet,**
   **daß** während der Umsetzung laufend ein Gemisch aus Reaktionsprodukten und Wasser abdestilliert, aus diesem gesamten Destillat Wasser ausgekreist und dann das trockene Produktgemisch destillativ aufgearbeitet wird.

5. Verfahren nach Anspruch 1,
   **dadurch gekennzeichnet,**

## EP 1 067 105 B1

**daß** die nur zum Teil umgesetzten Produkte 3-Chlorpropanol und Bis(3-chlorpropyl)ether in die Reaktion zurückgeführt werden.


## Claims

1. A process for the preparation of 1,3-dichloropropane,
   **characterized in that** it comprises
   reacting bis(3-hydroxypropyl) ether with hydrogen chloride in the presence of tertiary basic nitrogen compounds, distilling off the 1,3-dichloropropane which forms and water of reaction and working up both phases.

2. A process according to claim 1,
   **characterized in that**
   the reaction is carried out in the presence of pyridine, alkylpyridines quinoline, quinoline derivatives, dialkylanilines or trialkylamines or mixtures thereof.

3. A process according to claim 1,
   **characterized in that**
   the reaction takes place at a temperature of from 20°C to 190°C.

4. A process according to claim 1,
   **characterized in that**
   during the reaction a mixture of reaction products and water is continuously distilled off, water is removed azeotropically from this entire distillate and then the dry product mixture is worked up by distillation.

5. A process according to claim 1,
   **characterized in that** the only partially reacted products 3-chloropropanol and bis(3-chloropropyl) ether are returned to the reaction.


## Revendications

1. Procédé de préparation du 1,3-dichloropropane,
   **caractérisé en ce qu'**
   on fait réagir l'éther bis (3-hydroxypropylique) en présence de composés azotés basiques tertiaires, avec de l'acide chlorhydrique, on sépare par distillation le 1,3-dichloropropane qui s'est formé et l'eau de réaction et on traite les deux phases.

2. Procédé selon la revendication 1,
   **caractérisé en ce qu'**
   on effectue la réaction en présence de pyridine, d'alkylpyridines, de quinoléine, de dérivés de la quinoléine, de dialkylanilines ou de trialkylamines ou de leurs mélanges.

3. Procédé selon la revendication 1,
   **caractérisé en ce que**
   la réaction a lieu à une température comprise entre 20°C et 190°C.

4. Procédé selon la revendication 1,
   **caractérisé en ce qu'**
   on sépare par distillation d'une manière continue pendant la réaction un mélange à base de produit de réaction et d'eau, on évacue par circulation l'eau de ce distillat global, et ensuite on traite le mélange de produit séché par distillation.

5. Procédé selon la revendication 1,
   **caractérisé en ce qu'**
   on ramène les produits qut ont réagi seulement en partie - le 3-chloropropanol et l'éther bls(3-chloropropylique)- dans la réaction.